# EUROPEAN PATENT SPECIFICATION

(11) **EP 2 705 827 B1**
(45) Date of publication and mention of the grant of the patent: **27.12.2017**
(21) Application number: 12006385.4
(22) Date of filing: 11.09.2012
(51) Int. Cl.: A61K 6/083

(54) **Dental composition**
Dentale Zusammensetzung
Composition dentaire

(43) Date of publication of application: 12.03.2014
(73) Proprietor: DENTSPLY DETREY GmbH, 78467 Konstanz (DE)
(72) Inventor: Klee, Joachim E., Dr., 78315 Radolfzell (DE); Gansel, Julia, Dr., 78315 Radolfzell (DE); Fik, Christoph P., 78256 Steisslingen (DE); Wagner, Caroline, Dr., 78224 Singen (DE); Elsner, Oliver, Dr., 78476 Allensbach (DE); Maier, Maximilian, 40213 Düsseldorf (DE); Lampe, Ulrich, 40225 Düsseldorf (DE); Ritter, Helmut, Dr., 42111 Wuppertal (DE); Kavallar, Lisa-Maria, 46049 Oberhausen (DE)
(74) Representative: Hartz, Nikolai

(56) References cited:
- WO-A2-2008/015646
- US-A1- 2007 129 458

## Description

### Field of the Invention

The present invention relates to a dental composition comprising a specific polymerizable compound. Furthermore, the present invention relates to the use of the specific polymerizable compound for the preparation of a dental composition. The polymerizable compound of the present invention is a water soluble yet hydrolysis stable polyfunctional polymerizable monomer, which is copolymerizable with conventional (meth)acrylates and which has a low viscosity and excellent biocompatibility.

### Background of the Invention

Dental compositions containing polymerizable compounds are known. Conventionally, polymerizable dental compositions are provided for a broad range of applications and must, therefore, meet diverse requirements. For example, a polymerizable dental composition may be a dental adhesive composition, a bonding agent, a pit and fissure sealant, a dental desensitizing composition, a pulp capping composition, a dental composite, dental glass ionomer cement, a dental cement or a dental root canal sealer composition.

Conventionally, (meth)acrylates are used as polymerizable components in polymerizable dental compositions due to their excellent reactivity in radical polymerizations which may be demonstrated based on the polymerization enthalpy in the range of from Δ_{R}H = - 80 to 120 kJ/mol. In order to provide crosslinking capability, polyfunctional (meth)acrylates such as bis-GMA, were used for dental applications as early as 1962.

However, hydrolysis stability of (meth)acrylates is problematic in view of the acidity of many dental compositions which severely limits the storage stability of the dental composition. Moreover, hydrolysis taking place under biological conditions in the mouth of the patient is a further concern regarding (meth)acrylates.

EP1234543 discloses (meth)acrylamides having an increased hydrolysis stability. However, certain (meth)acrylamides have an increased viscosity as compared to the corresponding (meth)acrylates which is undesireable in the case of dental compositions wherein the polymerizable matrix is required to have a low viscosity.

WO 2008/015646 A2 discloses hair colouring and bleaching compositions.
US 2007/129458 A1 discloses dental compositions comprising bisacrylamides.

Allyl derivatives including allyl amine are usually not suitable as polymerizable compounds for dental compositions since the contribution of the allyl group to the heat of polymerization in a radical reaction is too low even in a copolymerization reaction with (meth)acrylates.

### Summary of the Invention

It is the problem of the present invention to provide a dental composition comprising a hydrolysis stable polyfunctional polymerizable monomer, which is copolymerizable with conventional (meth)acrylates and which has a low viscosity and excellent biocompatibility.

The present invention provides a dental composition comprising a polymerizable compound of the following formula (I):

A-L(B)ₙ (I)

wherein
- A: is a group of the following formula (II) 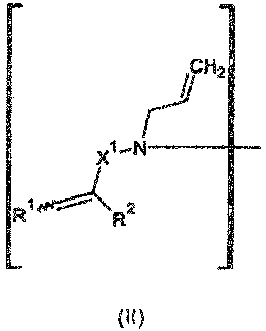
- X¹: is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10;
- R¹: is a hydrogen atom,
-COOM,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M,
a C₆₋₁₄ aryl or C₃₋₁₄ heteroaryl group which may be substituted by -COOM, -PO₃M, - O-PO₃M₂ or -SO₃M,
- R²: is a hydrogen atom,
-COOM
a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, or
a C₆₋₁₄ aryl or C₃₋₁₄ heteroaryl group which may be substituted by -COOM, -PO₃M, - O-PO₃M₂ and -SO₃M,
- L: is a linker group;
- B: independently is
a group according to the definition of A,
a group of the following formula (III) 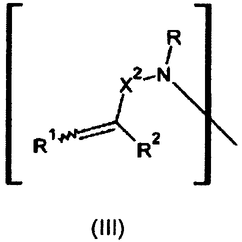 wherein
X² independently has the same meaning as defined for X¹ in formula (II),
R¹ and R² are independent from each other and independently have the same meaning as defined for formula (II),
R is a hydrogen atom,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, - O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M,
a C₆₋₁₄ aryl group which may be substituted by -COOM, -PO₃M, -O-PO₃M₂ or -SO₃M,
a group of the following formula (IV) 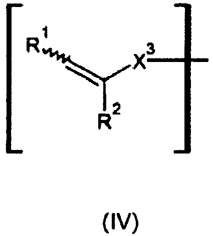 wherein
X³ is CO, -CH₂CO-, CS, or -CH₂CS-,
R¹ and R² which are independent from each other and independently have the same meaning as defined for formula (II), or a group [Z'X"]ₘE,
wherein
Z' is a straight chain or branched C₁₋₄ alkylene group,
X" is an oxygen atom, a sulfur atom or NH,
E is a hydrogen atom,
PO₃M₂,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, - PO₃M, -O-PO₃M₂ or -SO₃M,
a C₆₋₁₄ aryl or C₃₋₁₄ heteroaryl group which may be substituted by -COOM, -PO₃M, -O-PO₃M₂ or -SO₃M, and

m is an integer of from 1 to 10;
and
- n: is an integer of from from 1 to 4;
wherein M which are independent from each other each represent a hydrogen atom or a metal atom.

The present invention also provides a use of a compound of formula (I) for the preparation of a dental composition selected from a dental adhesive composition, a bonding agent, a pit and fissure sealant, a dental desensitizing composition, a pulp capping composition, a dental composite, dental glass ionomer cement, a dental cement and a dental root canal sealer composition.

The present invention is based on the recognition that a compound of formula (I) has a heat of polymerization which is comparable to the heat of polymerization of (meth)acrylates despite the presence of allyl groups. Moreover, the present invention is based on the recognition that the viscosity of the compounds of formula (I) is comparably low as compared to the corresponding (meth)acrylamides.

### Detailled Description of Preferred Embodiments

In this description, unless otherwise specified, a halogen atom denotes a fluorine atom, a chlorine atom, a bromine atom or a iodine atom; an alkyl group denotes, for example, a straight-chain or branched-chain C₁₋₁₆ alkyl group such as methyl, ethyl, propyl, isopropyl, butyl, sec-butyl, isobutyl, tert-butyl, pentyl, hexyl and octyll; a cycloalkyl group denotes a C₃₋₆ cycloalkyl group such as cyclopropyl, cyclobutyl, cyclopentyl and cyclohexyl; an aryl group denotes a C₆₋₁₄ aryl group such as phenyl, naphthyl; a heteroaryl group denotes a C₃₋₁₄ heteroaryl group which may contain 1 to 3 heteroatoms selected from nitrogen, oxygen and sulfur, such as furyl, thienyl, pyrrolyl, imidazolyl, pyrazolyl, thiazolyl, isothiazolyl, oxazolyl, isoxazolyl, thiadiazolyl, furazanyl, pyrrolidinyl, imidazolidinyl, pyridyl, pyrimidinyl, pyridzinyl, pyrazinyl, piperazinyl, piperidyl, morpholinyl, thiomorpholinyl, benzofuranyl, isobenzofuranyl, benzothienyl, indolinyl, isoindolinyl, indolizinyl, benzimidazolyl, benzoxazolyl, benzisoxazolyl, benzothiazolyl, benzoisothiazolyl, purinyl, coumarinyl, phthalazinyl, naphthyridinyl, quinoxalinyl, quinazolinyl, cinnolinyl, chromanyl, isochromanyl, chromenyl, thiochromenyl, quinuclidinyl, 1,3-benzodioxolyl, benzothiadiazolyl, benzoxadiazolyl, benzodioxanyl, quinolyl, isoquinolyl; an alkylene group denotes a C₁₋₄ alkylene group such as methylene, ethylene, propylene, butylene and hexylene.

The present invention provides a dental composition. The dental composition of the present invention is polymerizable or copolymerizable by a radical polymerization. The dental composition may be selected from a dental adhesive composition, a bonding agent, a pit and fissure sealant, a dental desensitizing composition, a pulp capping composition, a dental composite, dental glass ionomer cement, a dental cement and a dental root canal sealer composition.

A dental composition according to the present invention comprises a polymerizable compound of the following formula (I). The dental composition may comprise one or more compounds of formula (I). The dental composition of the present invention may comprise the polymerizable compound in an amount of from 0.5 to 99 percent by weight based on the total weight of the composition. Preferably, a dental adhesive composition comprises 2 to 50 percent by weight of one or more compounds of formula (I). Preferably, a bonding agent comprises 2 to 70 percent by weight of one or more compounds of formula (I). Preferably, a pit and fissure sealant comprises 2 to 80 percent by weight of one or more compounds of formula (I). Preferably, a dental desensitizing composition comprises 2 to 90 percent by weight of one or more compounds of formula (I). Preferably, a pulp capping composition comprises 2 to 50 percent by weight of one or more compounds of formula (I). Preferably, a dental composite comprises 2 to 50 percent by weight of one or more compounds of formula (I). Preferably, a dental glass ionomer cement comprises 2 to 50 percent by weight of one or more compounds of formula (I). Preferably, a dental cement comprises 2 to 50 percent by weight of one or more compounds of formula (I).

A compound of formula (I) is according to the following formula (I):

A-L(B)ₙ (I)

In formula (I), A is a specific polymerizable group which is linked by a single bond to a single group B or by a linking group to up to four groups B. In case more than one group B is present in the polymerizable compound of formula (I), the groups B may be the same or different. The groups B may be polymerizable.

According to the present invention, A is a group of the following formula (II)

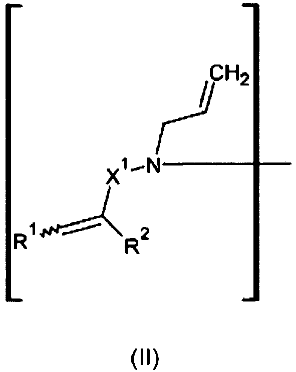

Accordingly, any compound of formula (I) is characterized by an allyl group bonded to a nitrogen atom and a further specific group having a polymerizable double bond which is bonded to the same nitrogen atom. The specific arrangement of the polymerizable double bond of the allyl group and a further polymerizable double bond which is bonded to the same nitrogen atom activates the allyl bond so that the allyl bond takes part in the radical polymerization reaction during curing. Given that an allyl group usually cannot be radically polymerized, it is surprising that the allyl group of a compound of the present invention contributes to the heat of polymerization of the polymerizable compound of formula (I) similar to a methacrylate group.

In formula (II), X¹ is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10.

In case X¹ is CO, CS, CH₂ in formula (II), a polymerizable double bond is present which may take part with the allyl group directly bonded to the nitrogen atom of the group of formula (II) in a cyclopolymerization reaction according to the following scheme.

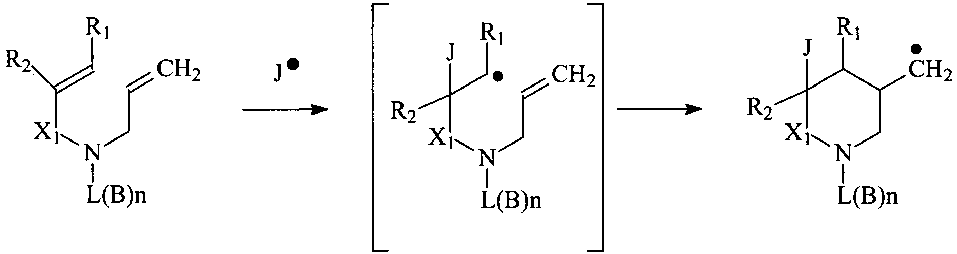

In case X¹ is a group [X'Z]ₖ in formula (II), it is preferred that L(B)ₙ provides a polymerizable double bond is present which may take part with the allyl group directly bonded to the nitrogen atom of the group of formula (II) in a cyclopolymerization reaction. Preferably, X' is an oxygen atom. Preferred examples for a straight chain or branched C₁₋₄ alkylene group for Z are an ethylene group and a propylene group. Preferably, k is an integer of from 1 to 4.

According to a preferred embodiment, X¹is CO.

In formula (II), R¹ is a hydrogen atom, -COOM, a straight chain or branched C₁₋₁₆ alkyl group which may be substituted by a C₃₋₆ cyctoalkyl group, a C₆₋₁₄ aryl or C₃₋₁₄ heteroaryl group, - COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or - SO₃M, a C₆₋₁₄ aryl or C₃₋₁₄ heteroaryl group which may be substituted by -COOM, -PO₃M, - O-PO₃M₂ or -SO₃M.

In formula (II), R¹ may be a straight chain or branched C₁₋₁₆ alkyl group, preferably a straight chain or branched C₁₋₁₆ alkyl group. Examples of the C₁₋₁₆ alkyl group of R¹ methyl, ethyl, n-propyl, i-propyl , n-butyl, isobutyl, tert-butyl sec-butyl, pentyl and hexyl. An aryl group may, for example, be a phenyl group or a naphthyl group. A C₃₋₁₄ heteroaryl group may contain 1 to 3 heteroatoms selected from nitrogen, oxygen and sulfur.

According to a preferred embodiment, R¹ is a hydrogen atom. In case R¹ is a C₁₋₆ alkyl group, the C₁₋₆ alkyl group is preferably substituted by -COOM.

In formula (II), R² is a hydrogen atom, -COOM, a straight chain or branched C₁₋₁₆ alkyl group which may be substituted by a C₆₋₁₄ aryl or C₃₋₁₄ heteroaryl group, -COOM, -PO₃M, -0-PO₃M₂ and -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, or a C₆₋₁₄ aryl or C₃₋₁₄ heteroaryl group which may be substituted by -COOM, -PO₃M, -O-PO₃M₂ and -SO₃M.

In formula (II), R² may be a straight chain or branched C₁₋₁₆ alkyl group, preferably a straight chain or branched C₁₋₆ alkyl group. Examples of the C₁₋₆ alkyl group of R² methyl, ethyl, n-propyl, i-propyl, n-butyl, isobutyl, tert-butyl sec-butyl, pentyl and hexyl. An aryl group may, for example, be a phenyl group or a naphthyl group. A C₃₋₁₄ heteroaryl group may contain 1 to 3 heteroatoms selected from nitrogen, oxygen and sulfur.

According to a preferred embodiment, R² is a hydrogen atom or a methyl group.

The following groups are preferred groups of formula (II), wherein M is a hydrogen atom or a metal atom:

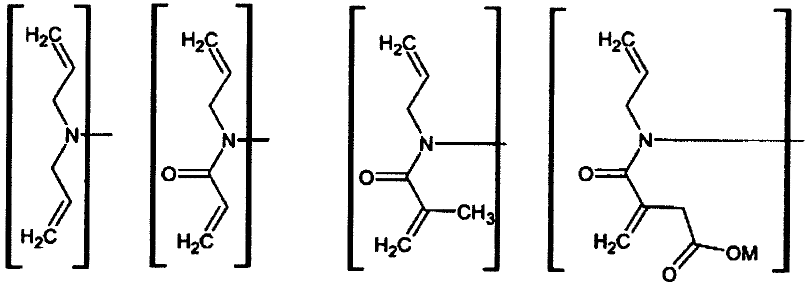

In formula (I), L is an (n+1) -valent linker group.

The linker group has a valency of at least two which corresponds to the total number of substituents A and B. Accordingly, L may be preferably divalent (n=2), trivalent (n=3), tetravalent (n=4), or pentavalent (n=5). Preferable, L is divalent or trivalent, most preferably divalent.

In general, L is a linear or branched monomeric, oligomeric, polymeric or copolymeric group. A monomeric groups is a low-molecular group having a molecular weight of up to 500. An oligomeric group is a group having a molecular weight of more than 500 to up to 10000 and may be prepared by a polymerization reaction. A polymeric or copolymeric group is a group having a molecular weight of more than 10000 which may be obtained by a polymerization reaction. The polymerization may be a condensation or addition reaction.

The linker group L may be a hydrocarbon group which may be aliphatic and/or aromatic. The hydrocarbon group may be substituted by 1 to 6 C₁₋₄ alkyl groups. Specific examples of the alkyl groups are methyl, ethyl, n-propyl, i-propyl, n-butyl, i-butyl or tert.-butyl. In a preferred embodiment, the hydrocarbon group of the linker group L may contain 1 to 5 oxygen atoms in the hydrocarbon group in the form of aliphatic or aromatic ether bonds, keto groups, carboxylic acid groups, or hydroxyl groups. Ester groups are not preferred in moiety L in view of hydrolysis stability of the polymerizable monomer. In case of an aliphatic group, L may be a straight chain or branched chain alkylene group or a cycloalkylene group. In case of an aromatic group, A may be an arylene group or a C₃₋₁₄ heteroarylene group. Specifically, L may be a divalent substituted or unsubstituted C₁ to C₂₀ alkylene group, substituted or unsubstituted C₆₋₁₄ arylene group, substituted or unsubstituted C₃ to C₂₀ cycloalkylene group, substituted or unsubstituted C₇ to C₂₀ arylenealkylenearylene group.

According to a preferred embodiment, L represents a saturated aliphatic C₂₋₂₀ hydrocarbon chain which may contain 2 to 4 oxygen atoms or nitrogen atoms, and which may be substituted by 1 to 6 C₁₋₆ alkyl groups, or L may be a substituted or unsubstituted C₇ to C₂₀ arylenealkylenearylene group which may be substituted by 1 to 6 C₁₋₄ alkyl groups.

In case L is an (n+1) -valent linker group, the linker group may preferably be a C₁₋₁₂ hydrocarbon group. The C₁₋₁₂ hydrocarbon group may contain 1 to 3 carbonyl groups or heteroatoms selected from oxygen, nitrogen and sulfur. Moreover, the C₁₋₁₂ hydrocarbon group may be substituted by a hydroxyl group, a C₆₋₁₄ aryl group, -COOM, -PO₃M, -O-PO₃M₂ or -SO₃M, wherein M is a hydrogen atom or a metal atom. Specific examples of a divalent C₁₋₁₂ hydrocarbon group are a straight chain or branched C₁₋₁₂ alkylene group such as a methylene, ethylene, propylene, butylene or penylene group which groups may be substituted by a hydroxyl group, a C₆₋₁₄ aryl group, -COOM, -PO₃M, -O-PO₃M₂ or -SO₃M.

In case L is an divalent linker group, the linker group may be polyamide residue of the following formula (V)

-Y-L'-Y'- (V)

wherein
Y and Y' represent CO and the polyamide moiety of formula (V) is obtainable by a process comprising the step of a step-growth polymerization of a mixture containing a diamine of the following formula (VI) and a compound of the following formula (VII) having at least two carboxylic acid groups,

R³(NHR")_{y} (VI)

wherein
- R³: represents an y-valent C₂₋₂₀ straight-chain, branched or cyclic hydrocarbon group which may optionally contain from 1 to 6 hetero atoms selected from nitrogen, oxygen, or sulphur atoms in the backbone of the hydrocarbon group, and optionally from 1 to 6 functional groups selected from hydroxyl groups, thiol groups and amino groups;
- R": represents a hydrogen atom, an allyl group, a straight chain or branched C₁₋₆ alkyl group which may be substituted by a C₃₋₆ cycloalkyl group, or a C₆₋₁₄ aryl group, a C₃₋₆ cycloalkyl group which may be substituted by a C₁₋₆ alkyl group or a C₆₋₁₄ aryl group; and
- y: represents an integer of at least 2;

MOOC-R⁴-COOM (VII)

wherein R⁴ represents a C₁₋₂₀ straight-chain, branched, cyclic or aromatic hydrocarbon group which may optionally contain a carbon-carbon double bond, from 1 to 6 heteroatoms selected from nitrogen, oxygen, or sulphur atoms in the backbone of the hydrocarbon group, and optionally from 1 to 6 functional groups selected from carboxylic acid groups, hydroxyl groups, thiol groups and amino groups, and the M which may be the same or different, independently represent a hydrogen atom or a metal atom.

Preferred divalent linker groups may be selected from methylene, ethylene, propylene, butylene and the following divalent groups:

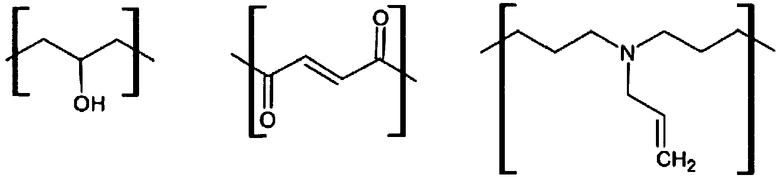

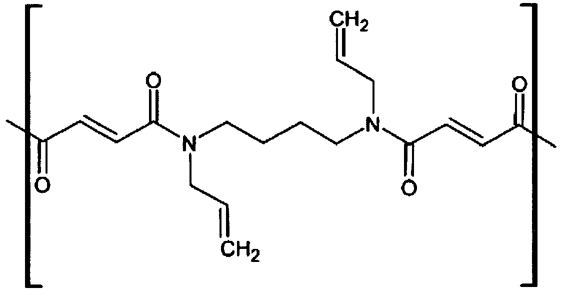

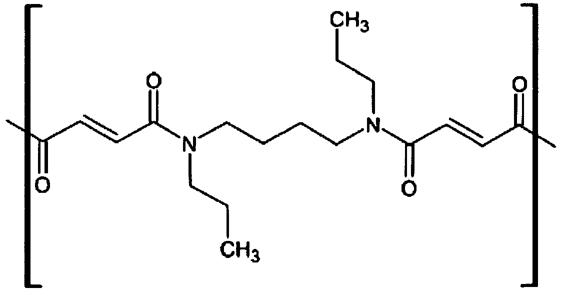

In formula (I), B independently is a group according to the definition of A, a group of the following formula (III), a group of the following formula (IV), or a group of the following formula [Z'X"]ₘE.

When B is a group according to formula (II) in the definition of A, then B may be the same or different from the group A present in a polymerizable compound of formula (I). Moreover, in case more than one group B according to the definition of A is present in the polymerizable compound of formula (I), the groups B may be the same or different. Specifically, B may be a group of the above formula (II) wherein an allyl group is bonded to a nitrogen atom, and a further group having a polymerizable double bond is bonded to the same nitrogen atom.

When B is a group according to formula (II) in the definition of A, X¹ is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10.

When B is a group according to formula (II) in the definition of A and in case X¹ is a group [X'Z]ₖ), it is preferred that L provides a polymerizable double bond which may take part with the allyl group directly bonded to the nitrogen atom of the group of formula (II) in a cyclopolymerization reaction. Preferably, X' is an oxygen atom. Preferred Examples for a straight chain or branched C₁₋₄ alkylene group for Z are an ethylene group and a propylene group. Preferably, k is an integer of from 1 to 4.

According to a preferred embodiment of B being a group according to formula (II) in the definition of A, X¹ is CO.

When B is a group according to formula (II) in the definition of A, R¹ is a hydrogen atom, - COOM, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or - SO₃M, a C₃₋₆ cycloalkyl group which may be substituted by a C₁₋₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M.

According to a preferred embodiment, R¹ is a hydrogen atom. In case R¹ is a C₁₋₆ alkyl group, the C₁₋₆ alkyl group is preferably substituted by -COOM.

When B is a group according to formula (II) in the definition of A, R² is a hydrogen atom, - COOM, a straight chain or branched C₁₋₁₆ alkyl group which may be substituted by a C₆₋₁₄ aryl group, -COOM, -PO₃M, -O-PO₃M₂ and -SO₃M, a C₃₋₆ cycloalkyl group which may be substituted by a C₁₋₁₆ alkyl group, a C₆₋₁₄ aryl group, -COOM, -PO₃M, -O-PO₃M₂ or -SO₃M, or a C₆₋₁₄ aryl or C₃₋₁₄ heteroaryl group which may be substituted by -COOM, -PO₃M, -O-PO₃M₂ and -SO₃M,

According to a preferred embodiment of B being a group according to formula (II) in the definition of A, R² is a hydrogen atom or a methyl group.

When B is a group of the formula (III), B is as follows.

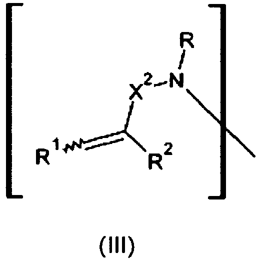

In formula (III), X² has the same meaning as defined for X¹ in formula (II). Moreover, in case more than one group X² according to the definition of X¹ is present in the polymerizable compound of formula (I), the groups X² may be the same or different. Specifically, When B is a group according to formula (III), X² is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10. X', Z and m of a group of formula (III) maybe the same or different of X', Z and k of any group of formula (II) present as A or B in the polymerizable compound of fromula (I).

When B is a group according to formula (III) and in case X² is a group [X'Z]ₖ), it is preferred that L provides a polymerizable double bond which may take part with the allyl group directly bonded to the nitrogen atom of the group of formula (II) in a cyclopolymerization reaction. Preferably, X' is an oxygen atom. Preferred Examples for a straight chain or branched C₁₋₄ alkylene group for Z are an ethylene group and a propylene group. Preferably, k is an integer of from 1 to 4.

According to a preferred embodiment of B being a group according to formula (III), X¹ is CO.

In formula (III), R¹ and R² are independent from each other and independently have the same meaning as defined for formula (II). Moreover, in case more than one group R¹ and R², respectively, is present when more than one group B of formula (III) is present in the polymerizable compound of formula (I), each of the groups R¹ and R² may be the same or different, respectively.

Specifically, when B is a group according to formula (III), R¹ is a hydrogen atom, -COOM, a straight chain or branched C₁₋₆ alkyl group which may be s ubstituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M.

According to a preferred embodiment when B is a group according to formula (III), R¹ is a hydrogen atom. In case R¹ is a C₁₋₁₆ alkyl group, the C₁₋₆ alkyl group is preferably substituted by -COOM.

When B is a group according to formula (III), R² is a hydrogen atom, -COOM, a straight chain or branched C₁₋₆ alkyl group which may be substituted by a C₆₋₁₄ aryl or C₃₋₁₄ heteroaryl group, -COOM, -PO₃M, -O-PO₃M₂ and -SO₃M, a C₃₋₆ cycloalkyl group which may be substituted by a C₁₋₆ alkyl group, a C₆₋₁₄ aryl or C₃₋₁₄ heteroaryl group, -COOM, - PO₃M, -O-PO₃M₂ or -SO₃M, or a C₆₋₁₄ aryl or C₃₋₁₄ heteroaryl group which may be substituted by -COOM, -PO₃M, -O-PO₃M₂ and -SO₃M,

According to a preferred embodiment of B being a group according to formula (III), R² is a hydrogen atom or a methyl group.

In formula (III), R is a hydrogen atom, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl group which may be substituted by -COOM, -PO₃M, -O-PO₃M₂ or -SO₃M.

Preferred groups of B of the formula (III) are as follows wherein M is a hydrogen atom o a metal atom.

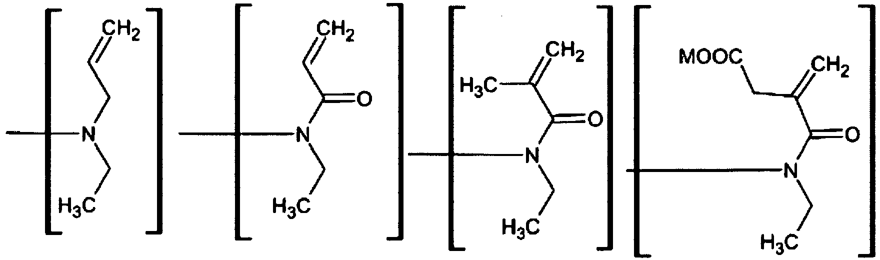

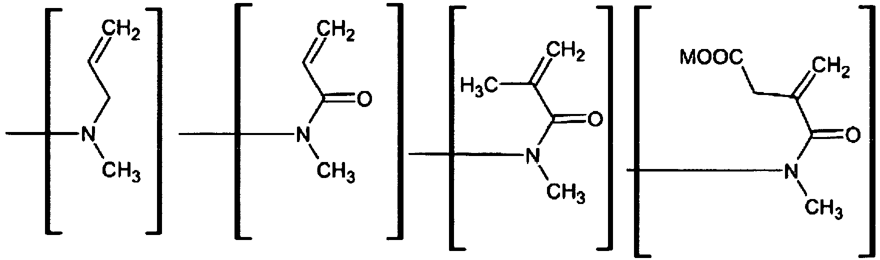

When B is a group of the formula (IV), B is as follows.

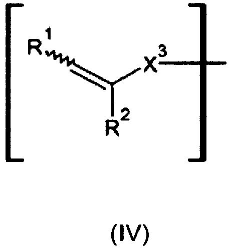

In formula (IV), X³ is CO, -CH₂CO-, CS, or -CH₂CS-,

According to a preferred embodiment, X³ is CO, -CH₂CO-.

In formula (IV), R¹ and R² are independent from each other and independently have the same meaning as defined for formula (II). Moreover, in case more than one group R¹ and R², respectively, is present when more than one group B of formula (IV) is present in the polymerizable compound of formula (I), each of the groups R¹ and R² may be the same or different, respectively.

Specifically, when B is a group according to formula (IV), R¹ is a hydrogen atom, -COOM, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M.

According to a preferred embodiment when B is a group according to formula (IV), R¹ is a hydrogen atom. In case R¹ is a C₁₋₁₆ alkyl group, the C₁₋₁₆ alkyl group is preferably substituted by -COOM.

When B is a group according to formula (IV), R² is a hydrogen atom, -COOM, a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M, a C₃₋₆ cycloalkyl group which may be substituted by a C₁₋₁₆ alkyl group, a C₆₋₁₄ aryl or C₃₋₁₄ heteroaryl group, -COOM, - PO₃M, -O-PO₃M₂ or -SO₃M, or a C₆₋₁₄ aryl group which may be substituted by -COOM, - PO₃M, -O-PO₃M₂ and -SO₃M,

According to a preferred embodiment of B being a group according to formula (IV), R² is a hydrogen atom or a methyl group.

Preferred groups of formula (IV) are as follows, whereby M is a hydrogen atom or a metal atom.

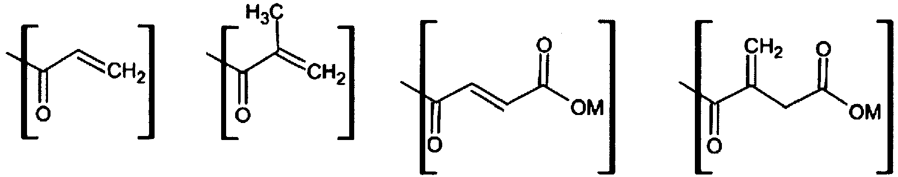

When B is a group [Z'X"]ₘE, the meaning of Z', X", m, and E is as follows.
t
Z' is a straight chain or branched C₁₋₄ alkylene group. Specific examples of the C₁₋₄ alkylene groups are methylene, ethylene, propylene and butylene.

X" is an oxygen atom, a sulfur atom or NH.

E is a hydrogen atom, PO₃M₂, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or-SO₃M.

m is an integer of from 1 to 10.

In formula (I), n is an integer of from from 1 to 4, preferably an integer of from 1 to 3. According to a first preferred generic group of compounds of formula (I), n is 1. According to a second preferred generic group of compounds of formula (I), n is 2.

According to the definition of the present invention, the M which are independent from each other, each represent a hydrogen atom or a metal atom. The metal atom is preferably an alkali metal or an alkaline earth metal. Specific examples of the alkali metal are lithium, sodium, and potassium. Specific examples of the alkaline earth metal are calcium, and magnesium.

The polymerizable compound of formula (I) may be prepared according to the following scheme, wherein A represent polymerizable moieties, B and C represent moieties which may form a linkage B-C. The linkage B-C may be formed by a condensation reaction or an addition reaction. The reaction may involve the formation of an amide bond, a urethane bond, and an ester bond. Preferably, the reaction involves the formation of an amide bond or a urethane bond. 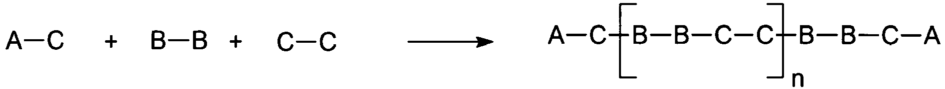 The preparation reaction may be carried out in a mixture of a compound having a single moiety C, a compound having two moieties B and a compound having two moieties C. Accordingly, a chain distribution of (B-B-C-C)n is observed whereby the certain positions such as the terminal positions, may be capped by a moiety A. Alternatively, it is possible to prepare a polymerizable compound of formula (I) by a reaction wherein a stoichiometric mixture of compound A-C and B-B is reacted and then the reaction product is reacted with a compound C-C. Alternatively, it is possible to prepare a polymerizable compound of formula (I) by a reaction wherein a mixture of compound B-B and C-C is reacted and then the reaction product is end-capped with A-C.

A specific example is as follows.

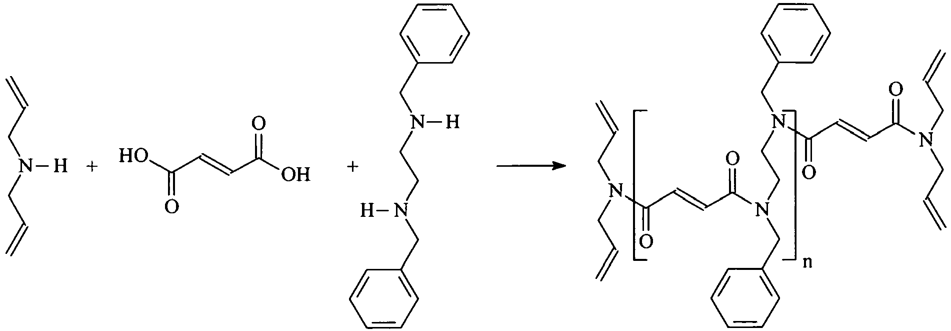

The reaction may be performed in accordance with, for example, the methods described in Jerry March "Advanced Organic Chemistry" Fourth Edition, John Wiley & Sons, INC., 1992 Richard C. Larock "Comprehensive Organic Transformation", VCH Publishers, INC., 1989 or a method conforming to the methods described above. The reaction may be carried out in a solvent which is capable of dissolving the reactants. An example of a suitable solvent is toluene. The reaction tempeature is not particularly limited. A suitable reaction temperature is from -30 °C to the boiling point of the solvent. The reaction time is not particularly limited and may be selected from 5 minutes to 48 hours.

### Initiator

The dental composition according to the present invention may comprise a polymerization initiator system. The initiator system may be based on a redox initiator or on a photoinitiator.

In case the dental composition contains a redox initiator, the amount of reducing agent and oxidizing agent should be sufficient to provide the desired degree of polymerization. Preferably, the mixed but unset cements of the invention contain a combined weight of about 0.01 to about 10%, more preferably about 0.2 to about 5%, and most preferably about 0.3 to about 3% of the reducing agent and oxidizing agent, based on the total weight (including water) of the mixed but unset cement components. The reducing agent or the oxidizing agent can be microencapsulated as described in U.S. Pat. No. 5,154,762. This will generally enhance shelf stability of the cement parts and if necessary permit packaging both the reducing agent and oxidizing agent together. Water-soluble and water-insoluble encapsulants can be employed. Suitable encapsulating materials include cellulosic materials as cellulose acetate, cellulose acetate butyrate, ethyl cellulose, hydroxymethyl cellulose and hydroxyethyl cellulose being preferred. Other encapsulants include polystyrene, copolymers of polystyrene with other vinylic monomers and polymethylmethacrylate, copolymers of methylmethacrylate with other ethylenically-unsaturated monomers. Preferred encapsulants are ethylcellulose and cellulose acetate butyrate. By varying the choice of encapsulant and the encapsulation conditions, the onset of curing can be tailored to start at times ranging from seconds to minutes. The ratio of amount of encapsulant to activator generally ranges from 0.5 to about 10 and preferably from about 2 to about 6.

Suitable oxidizing agents (initiators) include peroxides such as benzoyl peroxide cumene hydroperoxide (CHP), and tert-butyl hydroperoxide, ferric chloride, hydroxylamine (depending upon the choice of reducing agent), perboric acid and its salts, and salts of a permanganate or persulfate anion. Preferred oxidizing agents are peroxides, potassium persulfate, ammonium persulfate and hydrogen peroxide.

Suitable reducing agents (activators) include ascorbic acid, benzyl thiourea ferrous chloride, ferrous sulfate, hydrazine, hydroxylamine (depending upon the choice of oxidizing agent) oxalic acid, thiourea, and salts of a dithionite or sulfite anion. Preferred reducing agents include ascorbic acid and ferrous sulfate.

A photoinitiator should be capable of promoting polymerization of the polymerizable groups on exposure to light of a suitable wavelength and intensity. The photoinitiator preferably is sufficiently shelf-stable and free of undesirable coloration to permit its storage and use under typical dental conditions. Visible light photoinitiators are preferred. Suitable visible light-induced and ultraviolet light-induced initiators include an alpha-diketone (e.g., camphorquinone) with or without additional hydrogen donors (such as sodium benzene sulfinate, amines and amine alcohols). The photoinitiator may be present in an amount sufficient to provide the desired rate of photopolymerization. This amount will be dependent in part on the light source, the thickness of the cement layer to be exposed to radiant energy and the extinction coefficient of the photoinitiator. Preferably, mixed but unset photocurable cements of the invention will contain about 0.01 to about 5%, more preferably from about 0.1 to about 2% photoinitiator, based on the total weight (including water) of the mixed but unset cement components.

The invention will now be further explained based on the following examples:

### Example 1

### Preparation of 1,3-Bis-allylamino-2-propanol dihydrochloride salt

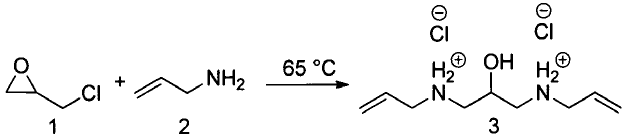

A 250 mL three-necked flask, equipped with septum and condenser, was charged with 75 mL Allylamine (1000 mmol) and was heated to 65 °C (oil bath temperature). Then, with continuous stirring, 19,6 mL Epichlorohydrin (250 mmol) were added dropwise over four hours and the mixture was stirred further for 16 hours under reflux. The white solid product was obtained by precipitation in acetone and has been washed with acetone to remove remaining byproducts and starting materials. The compound was evaporated under vacuum to dryness.

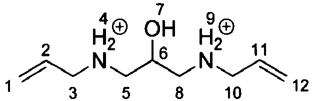

| | |
|---|---|
| **Yield:** | 14,5 g (24 % d.Th.) |
| **IR v [cm⁻¹]:** | 3290 (m), 3089 (w), 2947 (s), 2719 (s), 2431 (s), 1648 (w), 1447 (s), 1427 (m), 1113 (w), 992 (s), 931 (s) |
| **¹H-NMR [ppm]:** | (300 MHz, CDCl₃): 2.87-3.09 (m, 4H, H 5, 8), 3.57 (d, 4H, ³J_{H}= 6.82 Hz, H 3, 10), 4.27-4.31 (m, 1H, H 6), 5.38 (dd, 2H, ⁴J_{H}= 1.41 Hz, ³J_{H}= 10,39 Hz, H 1, 12), 5.47 (dd, 2H, ⁴J_{H}= 1.41 Hz, ³J_{H}= 17.19 Hz, H 1, 12), 5.87-6.00 (m, 2H, H 2, 11), 9,41 (s, 4 H, H 4, 9) |
| **MS (EI):** | m/z = 171 |

### 1.) Acrylation of 1,3-Bis-allylamino-2-propanol dihydrochloride salt

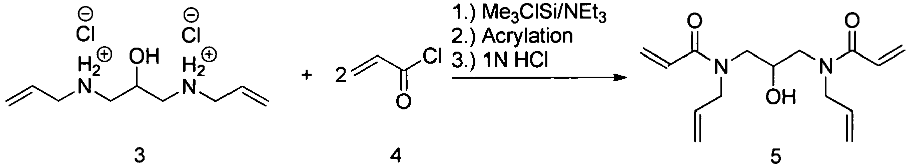

A 250 mL three-necked flask, equipped with septum and condenser, was charged with 2225 mg 1,3-Bis-allylamino-2-propanol dihydrochloride (9,18 mmol) solved in 100 mL Dichloromethane and 7,63 mL Triethylamine (101,2 mmol) under nitrogene atmosphere. The mixture was cooled to 5 °C and 4,1 mL Chlorotrimethylsilane (32,13 mmol) was added dropwise. After complete addition the reaction stirred overnight. Then, 1,6 mL Acryloyl chloride (20,2 mmol) solved in 20 mL dichloromethane was added dropwise keeping the temperature at -78 °C and stirred at room temperature for about 4 hours. After that, 100 mL 1N HCl was added and stirred for 2 hours. The organic layer was washed with 100 mL 1N HCl three times, dried over MgSO₄, filtered and evaporated under vacuum to dryness. The residue was purified by column chromatography eluting with CH₂Cl₂/EtOH (volume ratio, 95:5) to give a viscous liquid.

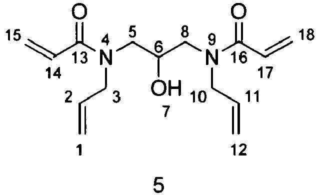

| | |
|---|---|
| **Yield:** | 1,52 g (59 % d.Th.) |
| **IR v [cm⁻¹]:** | 3370 (m), 3084 (w), 2927 (m), 1641 (s), 1605 (s), 1442 (s), 1417 (s), 1223 (s), 1101 (m), 795 (s) |
| **¹H-NMR [ppm]:** | (600 MHz, CDCl₃): 3.40-3.48 (m, 4H, H 5, 8), 4.04-4.07 (m, 4H, H 3, 10), 4.72 (d, 1H, H 6), 5.09 (d, 2H, H 1, 12), 5.16 (d, 2H, H 1, 12), 5.64 (d, 2H, H 15, 18), 5.70-5.78 (m, 2H, H 2, 11), 6.29 (d, 2H, H 15, 18), 6.42 (dd, 2H, H 14, 17) |
| **¹³C-NMR [ppm]:** | (150 MHz, CDCl₃): 51.90 (C 3, 10), 51.93 (C 5, 8), 71.80 (C 6), 117.07 (C 1, 12), 127.46 (C 2, 11), 129.00 (C 15, 18), 132.76 (C 14, 17), 168.55 (C 13, 16) |

The heat of polymerization of the compound of Example 1 is as follows:
D_{R}H= -129.7 ± 1.1 kJ/mol (37°C),
D_{R}H= -190.4 ± 21.0 kJ/mol (80°C), (p= 68.1%)

Considering that an acrylate group contributes to the heat of polymerization with about 80 kJ/mol, a compound of Example 1 would be expected to show a heat of polymerization of about 109 kJ/mol due to the presence of two acrylate groups reacting at a rate of 68.1 %. Accordingly, the remaining heat of polymerization of about 81 kJ/mol would be due to the allyl group. Given that usually, the allyl group does not take part in a radical polymerization, and given that in the present case each allyl group would contribute at a rate of 40.5 kJ/mol, a compound of the present invention provides reactive allyl groups which appear to contribute at a rate of 60 kJ/mol which is similar to a methacrylate group.

### Example 2 (Comparative)

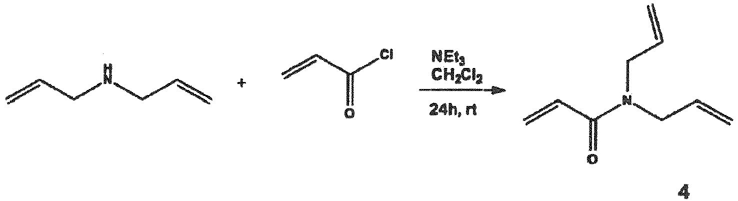

Compound **4** has been prepared by addition of acryloyl chloride and triethylamine to diallylamine in dichloromethane after stirring the mixture at room temperature for 24 hours. After vacuum distillation the product could be obtained in excellent purity, however, the yield of the reaction was 23 %.
After work-up by column chromatography (n-hexane : ethyl acetate, 90 : 10 to 75 : 25) 79 % of compound 4 could be obtained.
**¹H-NMR** (600 MHz, CDCl₃): *δ* [*ppm*] = 3.94 (*dd,* 2H, *J* = 4.4, 2.1 Hz, 3a-H), 4.04 (*dt,* 2H, *J* = 6.1, 1.4 Hz, 3b-H), 5.12-5.24 (*m,* 4H, 5a,b-H), 5.68 (dd, 1H, *J* = 10.4, 2.1 Hz, 1a-H), 5.75-5.83 (*m*, 2H, 4a,b-H), 6.37 (*dd,* 1H, *J* = 16.8, 2.1 Hz, 1b-H), 6.49 (*dd*, 1H, *J* = 16.7, 10.3 Hz, 2-H).
**IR** *^{v̂}*ₘₐₓ 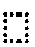 [*cm⁻¹*] = 3082 (w), 3012 (w), 2984 (w) 2916 (w), 1848 (w), 1649 (s, Amide I), 1613 (s), 1464 (m), 1437 (s), 1415 (s), 1360 (w), 1343 (w), 1277 (w), 1221 (s), 1193 (m), 1138 (w), 1059 (w), 979 (m), 956 (m), 920 (s), 794 (s), 686 (w), 653 (w), 558 (m), 508 (w).
**GC-MS** *^{m}*/*_{z}* (*l* in %) = 151 (8), 110 (25), 70 (25), 56 (55), 55 (100), 41 (25).

The heat of polymerization of the compound of Example 2 is as follows:
D_{R}H(37°C) = -135.1 ± 4.0 kJ/mol

The heat of polymerization indicates a reactivity of the allyl groups in a radical polymerization which is comparable to an acrylate group.

## Claims

1. Dental composition comprising a polymerizable compound of the following formula (I):
A-L(B)ₙ (I)
wherein
A is a group of the following formula (II) 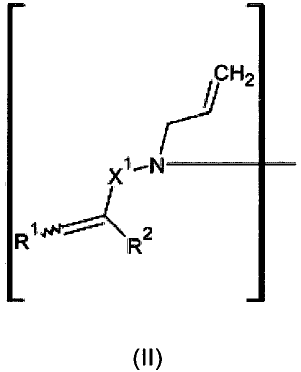
X¹ is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10;
R¹ is a hydrogen atom,
-COOM,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M,
a C₆₋₁₄ aryl or C₃₋₁₄ heteroaryl group which may be substituted by - COOM, -PO₃M, -O-PO₃M₂ or -SO₃M,
R² is a hydrogen atom,
-COOM
a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, or
a C₆₋₁₄ aryl or C₃₋₁₄ heteroaryl group which may be substituted by - COOM, -PO₃M, -O-PO₃M₂ and -SO₃M,
L is an (n+1) -valent linker group;
B independently is
a group according to the definition of A,
a group of the following formula (III) 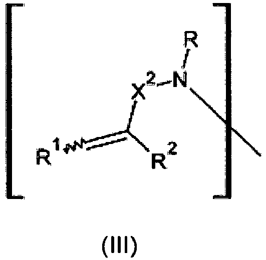 wherein
X² independently has the same meaning as defined for X¹ in formula (II),
R¹ and R² are independent from each other and independently have the same meaning as defined for formula (II),
R is a hydrogen atom,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, - PO₃M, -O-PO₃M₂ or -SO₃M,
a C₆₋₁₄ aryl or C₃₋₁₄ heteroaryl group which may be substituted by -COOM, -PO₃M, -O-PO₃M₂ or -SO₃M,
a group of the following formula (IV) 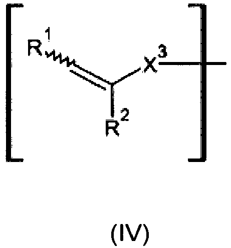 wherein
X³ is CO, -CH₂CO-, CS, or -CH₂CS-,
R¹ and R² which are independent from each other and independently have the same meaning as defined for formula (II), or
a group [Z'X"]ₘE, wherein
Z' is a straight chain or branched C₁₋₄ alkylene group,
X" is an oxygen atom, a sulfur atom or NH,
E is a hydrogen atom,
PO₃M₂,
a straight chain or branched C₁₋₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, - PO₃M, -O-PO₃M₂ or -SO₃M,
a C₆₋₁₄ aryl or C₃₋₁₄ heteroaryl group which may be substituted by -COOM, -PO₃M, -O-PO₃M₂ or -SO₃M, and
m is an integer of from 1 to 10; and
n is an integer of from from 1 to 4;
wherein M which are independent from each other each represent a hydrogen atom or a metal atom;
provided that in case L is a single bond, B cannot be a group according to the definition of A or a group of the formula (III).

2. The dental composition according to claim 1, wherein n is 1.

3. The dental composition according to any one of the preceding claims wherein X¹ is
CO.

4. The dental composition according to any one of claims 1, 2, or 3, wherein the linker group of L is a C₁₋₁₂ hydrocarbon group which may contain 1 to 3 carbonyl groups or heteroatoms selected from oxygen, nitrogen and sulfur, and which may be substituted by a hydroxyl group, a C₆₋₁₄ aryl group, -COOM, -PO₃M, -O-PO₃M₂ or-SO₃M, wherein M is a hydrogen atom or a metal atom.

5. The dental composition according to claim 4, wherein the C₁₋₁₂ hydrocarbon group is a group of the following formula (V), wherein
-Y-L'-Y'- (V)
wherein Y and Y' independently represent a single bond or CO or CS and L' is a C₁₋₁₂ alkylene group, a C₃₋₁₂ cycloalkylene group or a C₁₋₁₂ alkenylene group.

6. The dental composition according to claim 5, wherein X¹ is CO.

7. The dental composition according to claim 5, wherein Y is CO.

8. The dental composition according to claim 5, wherein R¹ is a hydrogen atom and R² is a hydrogen atom or a methyl group.

9. The dental composition according to claim 1, wherein the linker group is a polyamide residue of the following formula (V)
-Y-L'-Y'- (V)
wherein
Y and Y' represent CO and the polyamide moiety of formula (V) is obtainable by a process comprising the step of a step-growth polymerization of a mixture containing a diamine of the following formula (VI) and a compound of the following formula (VII) having at least two carboxylic acid groups,
R³(NHR")_{y} (VI)
wherein
R³ represents an y-valent C₂₋₂₀ straight-chain, branched or cyclic hydrocarbon group which may optionally contain from 1 to 6 hetero atoms selected from nitrogen, oxygen, or sulphur atoms in the backbone of the hydrocarbon group, and optionally from 1 to 6 functional groups selected from hydroxyl groups, thiol groups and amino groups;
R" represents a hydrogen atom, an allyl group a straight chain or branched C₁₋₆ alkyl group which may be substituted by a C₃₋₆ cycloalkyl group, or a C₆₋₁₄ aryl group, a C₃₋₆ cycloalkyl group which may be substituted by a C₁₋₆ alkyl group or a C₆₋₁₄ aryl group; and
y represents an integer of at least 2;
MOOC-R⁴-COOM (VII)
wherein R⁴ represents a C₁₋₂₀ straight-chain, branched, cyclic or aromatic hydrocarbon group which may optionally contain a carbon-carbon double bond, from 1 to 6 heteroatoms selected from nitrogen, oxygen, or sulphur atoms in the backbone of the hydrocarbon group, and optionally from 1 to 6 functional groups selected from carboxylic acid groups, hydroxyl groups, thiol groups and amino groups, and the M which may be the same or different, independently represent a hydrogen atom or a metal atom.

10. The dental composition according to any one of the preceding claims, wherein at least one of X¹ and Y is CO.

11. The dental composition according to any one of the preceding claims, wherein the compound of formula (I) has an average molecular weight of from 130 to 10,000, and/or which is selected from a dental adhesive composition, a bonding agent, a pit and fissure sealant, a dental desensitizing composition, a pulp capping composition, a dental composite, dental glass ionomer cement, a dental cement, a dental root canal sealer composition, and a dental infiltrant.

12. Use of a compound of formula (I) as defined in claim 1, for the preparation of a dental composition selected from a dental adhesive composition, a bonding agent, a pit and fissure sealant, a dental desensitizing composition, a pulp capping composition, a dental composite, dental glass ionomer cement, a dental cement, a dental root canal sealer composition, and a dental infiltrant.

## Patentansprüche

1. Dentalzusammensetzung, umfassend eine polymerisierbare Verbindung mit der folgenden Formel (I):
A-L(B)ₙ (I)
wobei
A eine Gruppe mit der folgenden Formel (II) ist, 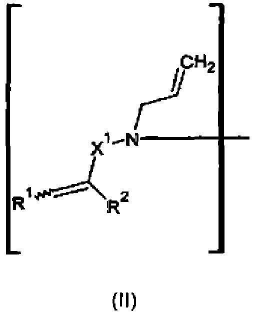
X¹ CO, CS, CH₂ oder eine Gruppe [X'Z]ₖ ist, wobei X' ein Sauerstoffatom, ein Schwefelatom oder NH ist, Z eine geradkettige oder verzweigte C₁₋₄-Alkylengruppe ist und k eine ganze Zahl von 1 bis 10 ist;
R¹ ein Wasserstoffatom,
-COOM,
eine geradkettige oder verzweigte C₁₋₁₆-Alkylgruppe, die mit einer C₃₋₆-Cycloalkylgruppe, einer C₆₋₁₄-Aryl- oder C₃₋₁₄-Heteroarylgruppe, -COOM, -PO₃M, -O-PO₃M₂ oder -SO₃M substituiert sein kann,
eine C₃₋₆-Cycloalkylgruppe, die mit einer C₁₋₁₆-Alkylgruppe, einer C₆₋₁₄-Aryl- oder C₃₋₁₄-Heteroarylgruppe, -COOM, -PO₃M, -O-PO₃M₂ oder -SO₃M substituiert sein kann,
eine C₆₋₁₄-Aryl- oder C₃₋₁₄-Heteroarylgruppe, die mit -COOM, -PO₃M, -O-PO₃M₂ oder -SO₃M substituiert sein kann, ist,
R² ein Wasserstoffatom,
-COOM,
eine geradkettige oder verzweigte C₁₋₁₆-Alkylgruppe, die mit einer C₆₋₁₄-Aryl- oder C₃₋₁₄-Heteroarylgruppe, -COOM, -PO₃M, -O-PO₃M₂ und -SO₃M substituiert sein kann,
eine C₃₋₆-Cycloalkylgruppe, die mit einer C₁₋₁₆-Alkylgruppe, einer C₆₋₁₄-Aryl- oder C₃₋₁₄-Heteroarylgruppe, -COOM, -PO₃M, -O-PO₃M₂ oder -SO₃M substituiert sein kann, oder
eine C₆₋₁₄-Aryl- oder C₃₋₁₄-Heteroarylgruppe, die mit -COOM, -PO₃M, -O-PO₃M₂ und -SO₃M substituiert sein kann, ist,
L eine (n+1)-wertige Verknüpfergruppe ist;
B unabhängig
eine Gruppe gemäß der Definition von A,
eine Gruppe mit der folgenden Formel (III), 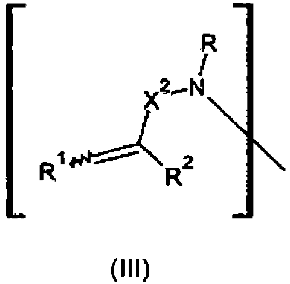 wobei
X² unabhängig die gleiche Bedeutung aufweist, wie für X¹ in Formel (II) definiert,
R¹ und R² unabhängig voneinander sind und unabhängig die gleiche Bedeutung aufweisen, wie für Formel (II) definiert,
R ein Wasserstoffatom,
eine geradkettige oder verzweigte C₁₋₁₆-Alkylgruppe, die mit einer C₃₋₆-Cycloalkylgruppe, einer C₆₋₁₄-Aryl- oder C₃₋₁₄-Heteroarylgruppe, -COOM, -PO₃M, -O-PO₃M₂ oder -SO₃M substituiert sein kann,
eine C₃₋₆-Cycloalkylgruppe, die mit einer C₁₋₁₆-Alkylgruppe, einer C₆₋₁₄-Aryl- oder C₃₋₁₄-Heteroarylgruppe, -COOM, -PO₃M, -O-PO₃M₂ oder -SO₃M substituiert sein kann,
eine C₆₋₁₄-Aryl- oder C₃₋₁₄-Heteroarylgruppe, die mit -COOM, -PO₃M, -O-PO₃M₂ oder -SO₃M substituiert sein kann, ist,
eine Gruppe mit der folgenden Formel (IV) 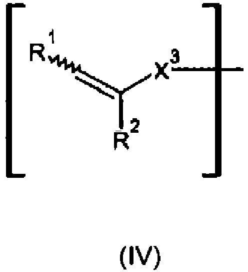 wobei
X³ CO, -CH₂CO-, CS oder -CH₂CS- ist,
R¹ und R², die unabhängig voneinander sind und unabhängig die gleiche Bedeutung aufweisen, wie für Formel (II) definiert, oder
eine Gruppe [Z'X"]ₘE ist,
wobei
Z' eine geradkettige oder verzweigte C₁₋₄-Alkylengruppe ist,
X" ein Sauerstoffatom, ein Schwefelatom oder NH ist,
E ein Wasserstoffatom,
PO₃M₂,
eine geradkettige oder verzweigte C₁₋₁₆-Alkylgruppe, die mit einer C₃₋₆-Cycloalkylgruppe,
einer C₆₋₁₄-Aryl- oder C₃₋₁₄-Heteroarylgruppe, -COOM, -PO₃M, -O-PO₃M₂ oder -SO₃M substituiert sein kann,
eine C₃₋₆-Cycloalkylgruppe, die mit einer C₁₋₁₆-Alkylgruppe, einer C₆₋₁₄-Aryl- oder C₃₋₁₉-Heteroarylgruppe, -COOM, -PO₃M, -O-PO₃M₂ oder -SO₃M substituiert sein kann,
eine C₆₋₁₄-Aryl- oder C₃₋₁₄-Heteroarylgruppe, die mit -COOM, -PO₃M, -O-PO₃M₂ oder -SO₃M substituiert sein kann, ist und m eine ganze zahl von 1 bis 10 ist; und
n eine ganze Zahl von 1 bis 4 ist;
wobei M, die unabhängig voneinander sind, jeweils ein Wasserstoffatom oder ein Metallatom darstellen;
mit der Maßgabe, dass, falls L eine Einfachbindung ist, B keine Gruppe gemäß der Definition von A oder Gruppe mit der Formel (III) sein kann.

2. Dentalzusammensetzung nach Anspruch 1, wobei n 1 ist.

3. Dentalzusammensetzung nach einem der vorhergehenden Ansprüche, wobei X¹ CO ist.

4. Dentalzusammensetzung nach einem der Ansprüche 1, 2 oder 3, wobei die Verknüpfergruppe L eine C₁₋₁₂-Kohlenwasserstoffgruppe ist, die 1 bis 3 Carbonylgruppen oder Heteroatome, ausgewählt aus Sauerstoff, Stickstoff und Schwefel, enthalten kann und die mit einer Hydroxylgruppe, einer C₆₋₁₄-Arylgruppe, -COOM, -PO₃M, -O-PO₃M₂ oder -SO₃M substituiert sein kann, wobei M ein Wasserstoffatom oder ein Metallatom ist.

5. Dentalzusammensetzung nach Anspruch 4, wobei die C₁₋₁₂-Kohlenwasserstoffgruppe eine Gruppe mit der folgenden Formel (V) ist,
-Y-L'-Y'- (V)
wobei Y und Y' unabhängig eine Einfachbindung oder CO oder CS darstellen und L' eine C₁₋₁₂-Alkylengruppe, eine C₃₋₁₂-Cycloalkylengruppe oder eine C₁₋₁₂-Alkenylengruppe ist.

6. Dentalzusammensetzung nach Anspruch 5, wobei X¹ CO ist.

7. Dentalzusammensetzung nach Anspruch 5, wobei Y CO ist.

8. Dentalzusammensetzung nach Anspruch 5, wobei R¹ ein Wasserstoffatom ist und R² ein Wasserstoffatom oder eine Methylgruppe ist.

9. Dentalzusammensetzung nach Anspruch 1, wobei die Verknüpfergruppe ein Polyamidrest mit der folgenden Formel (V) ist,
-Y-L'-Y'- (V)
wobei
Y und Y' CO darstellen und der Polyamid-Molekülteil von Formel (V) durch ein Verfahren erhältlich ist, das den Schritt einer Stufenwachstumspolymerisation eines Gemisches umfasst, das ein Diamin mit der folgenden Formel (VI) und eine Verbindung mit der folgenden Formel (VII), die mindestens zwei Carbonsäuregruppen aufweist, enthält,
R³(NHR")_{y} (VI)
wobei
R³ eine y-wertige geradkettige, verzweigte oder cyclische C₂₋₂₀-Kohlenwasserstoffgruppe darstellt, die wahlweise 1 bis 6 Heteroatome, ausgewählt aus Stickstoff-, Sauerstoff- oder Schwefelatomen, im Grundgerüst der Kohlenwasserstoffgruppe und wahlweise 1 bis 6 funktionelle Gruppen, ausgewählt aus Hydroxylgruppen, Thiolgruppen und Amingruppen, enthalten kann;
R" ein Wasserstoffatom, eine Allylgruppe,
eine geradkettige oder verzweigte C₁₋₆-Alkylgruppe, die mit einer C₃₋₆-Cycloalkylgruppe oder einer C₆₋₁₄-Arylgruppe substituiert sein kann,
eine C₃₋₆-Cycloalkylgruppe, die mit einer C₁₋₆-Alkylgruppe oder einer C₆₋₁₄-Arylgruppe substituiert sein kann, darstellt; und
y eine ganze Zahl von mindestens 2 darstellt;
MOOC-R⁴-COOM (VII)
wobei R⁴ eine geradkettige, verzweigte, cyclische oder aromatische C₁₋₂₀-Kohlenwasserstoffgruppe, die wahlweise eine Kohlenstoff-Kohlenstoff-Doppelbindung, 1 bis 6 Heteroatome, ausgewählt aus Stickstoff-, Sauerstoff- oder Schwefelatomen, im Grundgerüst der Kohlenwasserstoffgruppe und wahlweise 1 bis 6 funktionelle Gruppen, ausgewählt aus Carbonsäuregruppen, Hydroxylgruppen, Thiolgruppen und Amingruppen, enthalten kann, darstellt und die M, die gleich oder verschieden sein können, unabhängig ein Wasserstoffatom oder ein Metallatom darstellen.

10. Dentalzusammensetzung nach einem der vorhergehenden Ansprüche, wobei mindestens eines von X¹ und Y CO ist.

11. Dentalzusammensetzung nach einem der vorhergehenden Ansprüche, wobei die Verbindung mit der Formel (I) ein durchschnittliches Molekulargewicht von 130 bis 10.000 aufweist und/oder die aus einer dentalen Klebstoffzusammensetzung, einem Haftmittel, einem Grübchen- und Fissurenversiegelungsmittel, einem dentalen Desensibilisierungsmittel, einer Pulpaüberkappungszusammensetzung, einem Dentalkomposit, einem dentalen Glasionomerzement, einem Dentalzement, einer dentalen Wurzelkanal-Versieglerzusammensetzung und einem dentalen Infiltrationsmittel ausgewählt ist.

12. Benutzung einer Verbindung mit der Formel (I), wie in Anspruch 1 definiert, zur Herstellung einer Dentalzusammensetzung, ausgewählt aus einer dentalen Klebstoffzusammensetzung, einem Haftmittel, einem Grübchen- und Fissurenversiegelungsmittel, einer dentalen Desensibilisierungszusammensetzung, einer Pulpaüberkappungszusammensetzung, einem Dentalkomposit, einem dentalen Glasionomerzement, einem Dentalzement, einer dentalen Wurzelkanal-Versieglerzusammensetzung und einem dentalen Infiltrationsmittel.

## Revendications

1. Composition dentaire comprenant un composé polymérisable de la formule (I) suivante :
A-L(B)ₙ (I)
dans laquelle
A est un groupe de la formule (II) suivante : 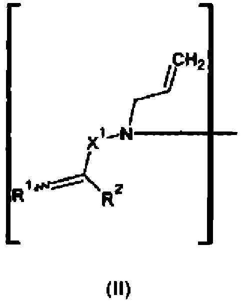
X¹ est CO, CS, CH₂ ou un groupe [X'Z]ₖ, dans laquelle X' est un atome d'oxygène, un atome de soufre ou NH, Z est un groupe alkylène en C₁₋₄ ramifié ou à chaîne droite et k est un nombre entier allant de 1 à 10 ;
R¹ est un atome d'hydrogène,
-COOM,
un groupe alkyle en C₁₋₁₆ ramifié ou à chaîne droite qui peut être substitué par un groupe cycloalkyle en C₃₋₆, un groupe aryle en C₆₋₁₄ ou hétéroaryle en C₃₋₁₄, -COOM,-PO₃M, -O-PO₃M₂ ou -SO₃M,
un groupe cycloalkyle en C₃₋₆ qui peut être substitué par un groupe alkyle en C₁₋₁₆ ou un groupe aryle en C₆₋₁₄ ou hétéroaryle en C₃₋₁₄, -COOM, -PO₃M, -O-PO₃M₂ ou -SO₃M,
un groupe aryle en C₆₋₁₄ ou hétéroaryle en C₉₋₁₄ qui peut être substitué par -COOM, -PO₃M, -O-PO₃M₂ ou -SO₃M,
R² est un atome d'hydrogène,
-COOM,
un groupe alkyle en C₁₋₁₆ ramifié ou à chaîne droite qui peut être substitué par un groupe aryle en C₆₋₁₄ ou hétéroaryle en C₃₋₁₄, -COOM, -PO₃M, -O-PO₃M₂ ou -SO₃M,
un groupe cycloalkyle en C₃₋₆ qui peut être substitué par un groupe alkyle en C₁₋₁₆ ou un groupe aryle en C₆₋₁₄ ou hétéroaryle en C₃₋₁₄, -COOM, -PO₃M, -O-PO₃M₂ ou -SO₃M, ou un groupe aryle en C₆₋₁₄ ou hétéroaryle en C₃₋₁₄ qui peut être substitué par -COOM, -PO₃M, -O-PO₃M₂ ou -SO₃M,
L est un groupe de liaison de valence (n + 1) ;
B est indépendamment
un groupe selon la définition de A,
un groupe de la formule (III) suivante 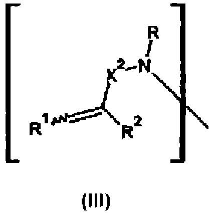 dans laquelle
X² a indépendamment la même signification que celle définie pour X¹ dans la formule (II),
R¹ et R² sont indépendants l'un de l'autre et ont indépendamment la même signification que celle définie pour la formule (II),
R est un atome d'hydrogène,
un groupe alkyle en C₁₋₁₆ ramifié ou à chaîne droite qui peut être substitué par un groupe cycloalkyle en C₃₋₆, un groupe aryle en C₆₋₁₄ ou hétéroaryle en C₃₋₁₄, -COOM,-PO₃M, -O-PO₃M₂ ou -SO₃M,
un groupe cycloalkyle en C₃₋₆ qui peut être substitué par un groupe alkyle en C₁₋₁₆ ou un groupe aryle en C₆₋₁₄ ou hétéroaryle en C₃₋₁₄, -COOM, -PO₃M, -O-PO₃M₂ ou -SO₃M,
un groupe aryle en C₆₋₁₄ ou hétéroaryle en C₃₋₁₄ qui peut être substitué par -COOM, -PO₃M, -O-PO₃M₂ ou -SO₃M,
un groupe de la formule (IV) suivante 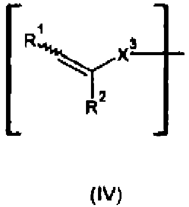 dans laquelle
X³ est CO, -CH₂CO-, CS ou -CH₂CS-,
R¹ et R² qui sont indépendants l'un de l'autre et ont indépendamment la même signification que celle définie pour la formule (II) ou
un groupe [Z'X"]ₘE,
dans laquelle
Z' est un groupe alkylène en C₁₋₄ ramifié ou à chaîne droite,
X" est un atome d'oxygène, un atome de soufre ou NH,
E est un atome d'hydrogène,
PO₃M₂,
un groupe alkyle en C₁₋₁₆ ramifié ou à chaîne droite qui peut être substitué par un groupe cycloalkyle en C₃₋₆, un groupe aryle en C₆₋₁₄ ou hétéroaryle en C₃₋₁₄, -COOM,-PO₃M, -O-PO₃M₂ ou -SO₃M,
un groupe cycloalkyle en C₃₋₆ qui peut être substitué par un groupe alkyle en C₁₋₁₆ ou un groupe aryle en C₆₋₁₄ ou hétéroaryle en C₃₋₁₄, -COOM, -PO₃M, -O-PO₃M₂ ou -SO₃M,
un groupe aryle en C₆₋₁₄ ou hétéroaryle en C₃₋₁₄ qui peut être substitué par -COOM, -PO₃M, -O-PO₃M₂ ou -SO₃M et
m est un nombre entier allant de 1 à 10 ; et
n est un nombre entier allant de 1 à 4 ;
dans laquelle M qui sont indépendants les uns des autres, représentent chacun un atome d'hydrogène ou un atome de métal ;
à condition que, dans le cas où L est une liaison simple, B ne puisse pas être un groupe selon la définition de A ou un groupe de la formule (III).

2. Composition dentaire selon la revendication 1, dans laquelle n est 1.

3. Composition dentaire selon l'une quelconque des revendications précédentes, dans laquelle X¹ est CO.

4. Composition dentaire selon l'une quelconque des revendications 1, 2 ou 3, dans laquelle le groupe de liaison de L est un groupe hydrocarboné en C₁₋₁₂ qui peut contenir de 1 à 3 groupes carbonyles ou des hétéroatomes sélectionnés parmi l'oxygène, l'azote et le soufre et qui peut être substitué par un groupe hydroxyle, un groupe aryle en C₆₋₁₄,-COOM, -PO₃M, -O-PO₃M₂ ou -SO₃M, dans laquelle M est un atome d'hydrogène ou un atome de métal.

5. Composition dentaire selon la revendication 4, dans laquelle le groupe hydrocarboné en C₁₋₁₂ est un groupe de la formule (V) suivante, dans laquelle
-Y-L'-Y'- (V)
dans laquelle Y et Y' représentent indépendamment une liaison simple ou CO ou CS et L' est un groupe alkylène en C₁₋₁₂, un groupe cycloalkylène en C₃₋₁₂ ou un groupe alkénylène en C₁₋₁₂.

6. Composition dentaire selon la revendication 5, dans laquelle X¹ est CO.

7. Composition dentaire selon la revendication 5, dans laquelle Y est CO.

8. Composition dentaire selon la revendication 5, dans laquelle R¹ est un atome d'hydrogène et R² est un atome d'hydrogène ou un groupe méthyle.

9. Composition dentaire selon la revendication 1, dans laquelle le groupe de liaison est un résidu de polyamide de la formule (V) suivante
-Y-L'-Y'- (V)
dans laquelle
Y et Y' représentent CO et la fraction de polyamide de la formule (V) peut être obtenue par un processus comprenant l'étape d'une polymérisation par croissance par étapes d'un mélange contenant une diamine de la formule (VI) suivante et un composé de la formule (VII) suivante ayant au moins deux groupes acide carboxylique,
R³(NHR")_{y} (VI)
dans laquelle
R³ représente un groupe hydrocarboné ramifié ou cyclique à chaîne droite en C₂₋₂₀ de valence y qui peut facultativement contenir de 1 à 6 hétéroatomes sélectionnés parmi les atomes d'azote, d'oxygène ou de soufre dans la chaîne principale du groupe hydrocarboné et, facultativement, de 1 à 6 groupes fonctionnels sélectionnés parmi les groupes hydroxyles, les groupes thiols et les groupes amino ;
R" représente un atome d'hydrogène, un groupe allyle un groupe alkyle en C₁₋₆ ramifié ou à chaîne droite qui peut être substitué par un groupe cycloalkyle en C₃₋₆, ou un groupe aryle en C₆₋₁₄,
un groupe cycloalkyle en C₃₋₆ qui peut être substitué par un groupe alkyle en C₁₋₆, ou un groupe aryle en C₆₋₁₄ ; et
y représente un nombre entier d'au moins 2 ;
MOOC-R⁴-COOM (VII)
dans laquelle R⁴ représente un groupe hydrocarboné ramifié, cyclique ou aromatique à chaîne droite en C₁₋₂₀ qui peut facultativement contenir une double liaison carbone-carbone, de 1 à 6 hétéroatomes sélectionnés parmi les atomes d'azote, d'oxygène ou de soufre dans la chaîne principale du groupe hydrocarboné et, facultativement, de 1 à 6 groupes fonctionnels sélectionnés parmi les groupes acide carboxylique, les groupes hydroxyles, les groupes thiols et les groupes amino et les M qui peuvent être les mêmes ou différents, représentent indépendamment un atome d'hydrogène ou un atome de métal.

10. Composition dentaire selon l'une quelconque des revendications précédentes, dans laquelle X¹ et/ou Y sont CO.

11. Composition dentaire selon l'une quelconque des revendications précédentes, dans laquelle le composé de la formule (I) présente un poids moléculaire moyen allant de 130 à 10 000 et/ou qui est sélectionné parmi une composition d'adhésif dentaire, un agent de liaison, une résine de scellement des puits et fissures, une composition de désensibilisation dentaire, une composition de coiffage de la pulpe, un composite dentaire, un ciment verre ionomère dentaire, un ciment dentaire, une composition de produit colmatant pour canal radiculaire et un infiltrant dentaire.

12. Utilisation d'un composé de la formule (I) telle que définie dans la revendication 1, pour la préparation d'une composition dentaire sélectionnée parmi une composition d'adhésif dentaire, un agent de liaison, une résine de scellement des puits et fissures, une composition de désensibilisation dentaire, une composition de coiffage de la pulpe, un composite dentaire, un ciment verre ionomère dentaire, un ciment dentaire, une composition de produit colmatant pour canal radiculaire et un infiltrant dentaire.
